# EUROPEAN PATENT SPECIFICATION

(11) **EP 1 051 964 B1**
(45) Date of publication and mention of the grant of the patent: **25.10.2006**
(21) Application number: 00107011.9
(22) Date of filing: 31.03.2000
(51) Int. Cl.: A61Q 19/00, A61K 8/60, A61K 8/31, A61K 8/34, A61K 8/89, A61K 8/92

(54) **Gommage cosmetic composition**
Kosmetische Zusammensetzung zum abreiben der Haut
Composition cosmétique de gommage

(30) Priority: 02.04.1999 JP 9639299
(43) Date of publication of application: 15.11.2000
(73) Proprietor: KAO CORPORATION, Tokyo 103-8210 (JP)
(72) Inventor: Koike, Kenzo, Kao Corporation, Research Laborat., Tokyo 131-8501 (JP); Ueno, Kaori, Kao Corporation, Research Laborat., Tokyo 131-8501 (JP); Shimizu, Masaki, Kao Corporation, Research Laborat, Tokyo 131-8501 (JP); Ohta, Hiroshi, Kao Corporation, Research Laborat., Tokyo 131-8501 (JP)
(74) Representative: HOFFMANN EITLE

(56) References cited:
- EP-A- 0 950 400
- WO-A-89/03669
- US-A- 3 803 041
- US-A- 4 626 550
- DATABASE WPI Derwent Publications Ltd., London, GB; AN 1999604893 XP002157146 & JP 11 263721 A (SHISEIDO), 28 September 1999 (1999-09-28)
- DATABASE CHEMABS [Online] CHEMICAL ABSTRACTS SERVICE, COLUMBUS, OHIO, US; Database accession no. 1996:50739 XP002157144 & JP 07 268247 A (T. SAITO ET AL.) 17 October 1995 (1995-10-17)
- DATABASE CHEMABS [Online] CHEMICAL ABSTRACTS SERVICE, COLUMBUS, OHIO, US; Database accession no. 1995:375050 XP002157145 & JP 06 329525 A (KAO) 29 November 1994 (1994-11-29)
- PATENT ABSTRACTS OF JAPAN vol. 15, no. 7 (C-0794), 9 January 1991 (1991-01-09) & JP 02 258713 A (SHISEIDO), 19 October 1990 (1990-10-19)
- PATENT ABSTRACTS OF JAPAN vol. 1999, no. 01, 29 January 1999 (1999-01-29) & JP 10 279470 A (NICHIDEN KAGAKU), 20 October 1998 (1998-10-20)
- DATABASE WPI Derwent Publications Ltd., London, GB; AN 1996368557 XP002157147 & JP 08 176599 A (KANEBO), 9 July 1996 (1996-07-09)
- PATENT ABSTRACTS OF JAPAN vol. 16, no. 375 (C-0973), 12 August 1992 (1992-08-12) & JP 04 120015 A (KANSAI KOUSO), 21 April 1992 (1992-04-21)

## Description

### BACKGROUND OF THE INVENTION

### Field of the Invention:

The present invention relates to a *gommage* cosmetic composition which can form *gommage* (eraser refuse-like fine particles) on the wet skin, scarcely becomes a burden to the skin, has an excellent effect by the *gommage* formed, and gives users a pleasant feeling upon use.

### Description of the Background Art:

The conventional *gommage* cosmetic compositions are used on the dry skin free of water and serve to remove dirt on the skin and an aged horny layers by crumbled fine particles having a particle size of about 0.5 to 5 mm like eraser refuse, which are formed by evaporation of solvent contained in the cosmetic compositions by applying and massaging (Japanese Patent Application Laid-Open No. 287542/1998). A great number of such cosmetic compositions have been already marketed as *gommage* type or peeling type cosmetics (for example, Pretty, trade name; product of White Japan Corporation).

These *gommage* cosmetic compositions involve problems that they become a great burden to the skin because the skin is strongly rubbed due to their use on the dry skin as described above, and that refuse falls down on the floor, resulting in difficulty in disposing of it. When the treatment with such a *gommage* cosmetic composition is applied in a bathroom where the refuse is easy to be cleaned up, there is also offered a problem that *gommage* is hard to be formed due to high humidity.

### SUMMARY OF THE INVENTION

It is an object of the present invention to provide a *gommage* cosmetic composition which scarcely becomes a burden to the skin, permits forming *gommage* with ease and has an excellent effect to remove dirt on the skin and an aged horny layers.

The present inventors have found that a cosmetic composition containing a specific water-soluble polymer and nonaqueous solvent forms *gommage* on the wet skin, scarcely becomes a burden to the skin because water gives the skin a lubricating action, can efficiently remove dirt on the skin and an aged horny layers, has an excellent effect by the *gommage* formed, can make the skin smooth in a short period of time and gives users a pleasant feeling upon use. It has also been found that when a component which generates heat upon contact with water is contained in such a cosmetic composition, it gives users an excellent warmed feeling and has an excellent effect by the *gommage* formed at the same time. It has been further found that when a surfactant is contained in this cosmetic composition, a detergent having foamability, a cleansing effect and an effect by the *gommage* formed in combination can be provided.

According to the present invention, there is thus provided a *gommage* cosmetic composition comprising (A) a water-soluble polymer selected from modified starch and triglucopolysaccharide, soluble starch, dextrin, cyclodextrin, maltodextrin and hydrolysed and reduced products of starch and (B) a substance that is liquid at 25°C (excluding water) selected from polyols, lower alcohols, liquid paraffin, silicone oil and oils and fats and wherein the water content of the composition is at most 5% by weight.

According to the present invention, there is also provided a *gommage* cosmetic composition comprising (A) a water-soluble polymer selected from modified starch and triglucopolysaccharide, soluble starch, dextrin, cyclodextrin, maltodextrin and hydrolysed and reduced products of starch (B) a substance that is liquid at 25°C (excluding water) selected from polyols, lower alcohols, liquid paraffin, silicone oil and oils and fats, and (C) a component which generates heat upon contact with water and wherein the water content of the composition is at most 5% by weight.

According to the present invention, there is further provided a *gommage* cosmetic composition comprising (A) a water-soluble polymer selected from modified starch and triglucopolysaccharide, soluble starch, dextrin, cyclodextrin, maltodextrin and hydrolysed and reduced products of starch, (B) a substance that is liquid at 25°C (excluding water) selected from polyols, lower alcohols, liquid paraffin, silicone oil and oils and fats, and (D) a surfactant and wherein the water content of the composition is at most 5% by weight.

According to the present invention, there is still further provided a *gommage* cosmetic composition comprising (A) a water-soluble polymer selected from modified starch and triglucopolysaccharide, soluble starch, dextrin, cyclodextrin, maltodextrin and hydrolysed and reduced products of starch, (B) a substance that is liquid at 25°C (excluding water) selected from polyols, lower alcohols, liquid paraffin, silicone oil and oils and fats (C) a component which generates heat upon contact with water, and (D) a surfactant and wherein the water content of the composition is at most 5% by weight.

The cosmetic compositions according to the present invention can form *gommage* on the wet skin, scarcely become a burden to the skin, have an excellent effect by the *gommage* formed, and give users a pleasant feeling upon use.

The above and other objects, features and advantages of the present invention will be readily appreciated as the same becomes better understood from the preferred embodiments of the present invention, which will be described subsequently in detail, and from the appended claims.

### DETAILED DESCRIPTION OF THE PREFERRED EMBODIMENTS

Component (A) used in the present invention can form *gommage* when it is rubbed on the skin in a state mixed with water. However, a water-soluble polymer which forms *gommage* when a 20 % by weight solution of the water-soluble polymer in polyethylene glycol (average molecular weight: 400) is rubbed on the skin in a state mixed with the same weight of water is preferred.

Component (A) is chosen from modified starch and triglucopolysaccharides, with modified starch and triglucopolysaccharides having a weight average molecular weight of 500 to 200,000 being preferred from the viewpoints of easy availability and the ability to form *gommage*. Further, soluble starch, dextrin, cyclodextrin, maltodextrin, hydrolyzed and reduced products of starch and triglucopolysaccharides are particularly preferred.

These water-soluble polymers (A) may be used either singly or in any combination and are preferably contained in a proportion of 1 to 80 % by weight, particularly 8 to 50 % by weight, more particularly 10 to 30 % by weight based on the total weight of the composition in that a far excellent effect by *gommage* formed is brought about.

Component (B) is selected from polyols such as polyethylene glycol (average molecular weight: 200, 300, 400, 600, etc.), 1,3-butylene glycol, propylene glycol, dipropylene glycol and glycerol; lower alcohols such as ethanol and propanol; hydrophobic solvents such as liquid paraffin and silicone oil; oils and fats such as triolein.

These nonaqueous solvents may be used either singly or in any combination thereof and are preferably contained in a proportion of 10 to 80 % by weight, particularly 30 to 70 % by weight, more particularly 50 to 70 % by weight based on the total weight of the composition from the viewpoint of the ability to form *gommage.*

The cosmetic composition according to the present invention is substantially free of water so as not to prevent the formation of *gommage*, since the *gommage* is formed by adding water from the outside, with a nonaqueous system that scarcely contains water being particularly preferred. The term "substantially free of water" as used herein means that the composition contains water in a proportion of at most 5 % by weight.

The cosmetic composition according to the present invention may comprise (C) a component which generates heat upon contact with water for the purpose of giving users a warmed feeling in addition to the effect by the *gommage*. Examples of the component (C) include solid heat-generating substances such as activated zeolite, various kinds of inorganic salts, silica gel and activated alumina; and liquid heat-generating substances such as polyethylene glycol (average molecular weight: 200, 400, 600, etc.), glycerol and polyoxyethylene methylglucoside. Examples of the inorganic salts used herein include sulfates such as magnesium sulfate (MgSO₄, MgSO₄·H₂O, MgSO₄·4H₂O), aluminum sulfate (Al₂(SO₄)₃) and calcium sulfate (CaSO₄, CaSO₄·1/2H₂O, CaSO₄·2H₂O); chlorides such as calcium chloride (CaCl₂, CaCl₂·H₂O, CaCl₂·2H₂O), magnesium chloride (MgCl₂, MgCl₂·2H₂O, MgCl₂·4H₂O) and aluminum chloride (AlCl₃, AlCl₃·6H₂O); and besides calcium aluminate, dry alum, calcium oxide, sodium carbonate and sodium hydrogenphosphate.

No particular limitation is imposed on the activated zeolite. However, zeolite A-3, A-4 and A-5 represented by the formula Na₂O·Al₂O₃·2SiO₂·ZH₂O (Z denoting any number) are preferred from the viewpoints of easy availability, profitability, etc. Zeolite subjected to a special treatment such as ion exchange or neutralizing treatment may also be used.

Among these heat-generating components, activated zeolite, magnesium chloride, magnesium sulfate, calcium aluminate, polyethylene glycol and glycerol are particularly preferred.

These heat-generating components (C) may be used either singly or in any combination thereof and are preferably contained in a proportion of at least 10 % by weight, particularly 10 to 60 % by weight, more particularly 20 to 40 % by weight based on the total weight of the composition. Among these heat-generating components (C), the liquid heat-generating components may overlap the component (B). In this case, such a component serves both as the solvent of the component (A) and as the heat-generating component (C).

The cosmetic composition according to the present invention may further comprise (D) a surfactant for the purpose of imparting both foaming and detergent effects to the resulting composition. As the surfactant (D), is preferred that having foamability and cleansability, and an anionic, nonionic or amphoteric surfactant is more preferred, with an anionic or nonionic surfactant being particularly preferred.

Examples of the anionic surfactant include acylisethionates, higher fatty acid salts, acylamino acid salts, monoalkylphosphates, alkylsulfonates, polyoxyalkylene alkyl ether sulfates and alkylbenzenesulfonates. The acyl or alkyl groups in these anionic surfactants preferably have 6 to 24 carbon atoms. Among these anionic surfactants, C₆-C₂₆-acylisethionates, higher fatty acid sodium salts, N-acyl-β-alanine salts (for example, N-lauroyl-β-alanine sodium salt), N-acylsarcosinates (for example, sodium lauroylsarcosinate) and mono-C₆-C₂₆-alkylphosphates.

Examples of the nonionic surfactant include fatty acid monoethanolamides, alkylglycosides, monoglycerides and diglycerides, and specific examples thereof include C₆-C₂₆ fatty acid monoethanolamides and C₆-C₂₆₋alkylglycosides.

Examples of the amphoteric surfactant include betaine type surfactants and amino acid type surfactants.

These surfactants may be used either singly or in any combination thereof and are preferably contained in a proportion of 1 to 80 % by weight, particularly 5 to 50 % by weight, more particularly 10 to 30 % by weight based on the total weight of the composition from the viewpoints of foamability and cleansability.

Combined use of both component (C) and component (D) in the cosmetic composition according to the present invention is particularly preferred, since both warming effect and cleansing effect are brought about.

When water-insoluble or hardly water-soluble powder is further contained in the *gommage* cosmetic compositions according to the present invention, the formation of *gommage* particles can be facilitated. Examples of such powder include inorganic powders such as kaolin, bentonite, alumina and titanium oxide; hardly water-soluble polysaccharides such as crystalline cellulose; and organic powders such as polyethylene beads and silicone powder.

These powders may be used either singly or in any combination thereof and are preferably contained in a proportion of 10 to 50 % by weight, particularly 5 to 40 % by weight, more particularly 10 to 30 % by weight based on the total weight of the composition from the viewpoints of easy formation of *gommage* particles and the achievement of a higher effect by *gommage* formed.

The *gommage* cosmetic compositions according to the present invention can be prepared by incorporating an oil component that is solid at 25°C, a moisturizer, a ultraviolet absorbent, a thickener, a stabilizer, a perfume base, coloring matter, etc. in addition to the above-described component in accordance with a method known *per se* in the art.

The *gommage* cosmetic compositions according to the present invention may be prepared in any form such as lotion, cream, paste or solid by selecting the kinds and amounts of the components blended. A composition having a low viscosity (5 Pa·s or lower) like liquid or lotion is preferred for a wide area, for example, the whole body, while a composition having a comparatively high viscosity (10 Pa·s or higher) is preferred for a part (for example, an elbow, knee, etc.) of the body, or a part where sags and runs are not preferred, such as the face.

The cosmetic composition according to the present invention is suitable for use as massaging compositions, packs or detergents. When the cosmetic composition is used for massage, it is only necessary to apply it to the wet or dry skin to massage the skin. If the skin is wet, *gommage* is formed before long, and a scrubbed feeling is also given together with the effect by the *gommage* upon a massage. When a surfactant is incorporated into the composition at this time, the composition also functions as a detergent. When the composition is applied to the dry skin, a massage can be sufficiently given, and the effect by *gommage* is exhibited during the massage or by adding water later.

When the composition is used as a pack, it is applied to the wet or dry skin and left to stand for a while. *Gommage* is formed, since a moderate amount of water is added when the composition is washed out with water while lightly rubbing the skin, so that the effect by the *gommage* is brought about.

The *gommage* formed by using the cosmetic composition according to the present invention is dissolved or dispersed in a great amount (for example, ten times as much as the *gommage* formed) of water. Accordingly, even when the cosmetic composition is used in a bathroom or the like, a drainageway is prevented from clogging by washing out the composition with a sufficient amount of water, so that the composition can be used safely.

### Example 1:

*Gommage* cosmetic compositions having their corresponding formulations shown in Table 1 were prepared in accordance with a method known *per se* in the art to evaluate them as to a feeling upon use. The results are shown collectively in Table 1.

### (Evaluation method)

Each of the cosmetic compositions was applied to the wet skins (upper arms) of 10 Japanese women panelists of their twenties to thirties after a bath to massage them, thereby evaluating the cosmetic compositions as to an effect by *gommage* (on the wet skin), burden to the skin, smoothness of the skin (after use) and a feeling upon use (massaged feeling) in accordance with the following standard.

### (Evaluation standard)

- ⊚:: At least 8 panelists of 10 panelists judged to be good;
- ○:: At least 6 panelists of 10 panelists judged to be good;
- △:: At least 3 panelists of 10 panelists judged to be good;
- ×:: At most 2 panelists of 10 panelists judged to be good.

**Table 1**

| Component (% by weight) | Product 1 | Invention product 2 | Comparative product |
|---|---|---|---|
| (Water-soluble polymer) | | | |
| Starch hydrolyzate (molecular | 30 | 0 | 0 |
| weight: 1000) | | | |
| α-Cyclodextrin | 0 | 10 | 0 |
| (Nonaqueous solvent) | | | |
| Glycerol | 3 | 3 | 3 |
| Polyethylene glycol 400 | 40 | 40 | 40 |
| 1,3-Butylene glycol | 10 | 10 | 10 |
| Liquid paraffin | 3 | 3 | 3 |
| Polyethylene glycol monolaurate | 1 | 1 | 1 |
| Ethanol | 2 | 5 | 5 |
| (Water-insoluble or hardly | | | |
| water-soluble powder) | | | |
| Kaolin | 0 | 15 | 15 |
| Crystalline cellulose (water- | 0 | 10 | 20 |
| insoluble) | | | |
| (Other components) | | | |
| Thickener | 0.5 | 0.5 | 0.5 |
| Purified water | Balance | Balance | Balance |
| Perfume base | q.s. | q.s. | q.s. |
| Total | 100 | 100 | 100 |
| Effect by *gommage* (on the wet skin) | ⊚ | ⊚ | × (no *gomnage was formed)* |
| Burden to the skin | ○ | ○ | - |
| Smoothness of the skin (after use) | ⊚ | ○ | △ |
| Feeling upon use (massaged feeling) | ○ | ⊚ | △ |

### Example 2:

A cosmetic composition having a formulation shown below was produced in accordance with a method known *per se* in the art.

| (Components) | (% by weight) |
|---|---|
| Starch hydrolyzate (dextrin; average molecular weight: 2000) | 15 |
| 1,3-Butylene glycol | 20 |
| Dipropylene glycol | 10 |
| Polyoxyethylene methylglucoside | 20 |
| Glycerol | 4 |
| Liquid paraffin | 3 |
| Kaolin | 20 |
| Aluminum oxide | 3 |
| Polyoxyethylene·methyl polysiloxane copolymer | 1 |
| Perfume base | q.s. |
| Thickener, stabilizer | q.s. |
| Purified water | Balance |
| Total | 100 |

The cosmetic composition thus obtained, and a marketed *gommage* cosmetic composition were evaluated as to a feeling upon use in the same manner as in Example 1. The results are shown in Table 2.

**Table 2**

| Conditions for use | | Invention product 3 | Marketed product |
|---|---|---|---|
| | | Wet skin | Dry skin |
| Feeling upon use | Formation of *gommage* (dry skin) | - | ○ |
| | Formation of *gommage* (wet skin) | ⊚ | × |
| | Feel of *gommage* against the skin (liking) | ○ | △ |
| | Remained feeling of powder | ⊚ | △ |
| | Burden to the skin | ○ | × |
| | Slippery feeling (present) | ⊚ | ○ |
| | Slick feeling (present) | ⊚ | △ |
| | Moisturized feeling (present) | ⊚ | × |
| | Smooth feeling (present) | ○ | ○ |

### Example 3:

*Gommage* cosmetic compositions having their corresponding formulations shown in Table 3 were prepared in accordance with a method known *per se* in the art to evaluate them as to an effect by *gommage* and a warmed feeling upon their use in accordance with the same evaluation standard as in Example 1.

As a result, it is understood that when a heat-generating component is incorporated into the cosmetic compositions according to the present invention, a good warmed feeling is given in addition to the effect by *gommage* as shown in Table 3.

**Table 3**

| (% by weight) | | | |
|---|---|---|---|
| | Invention product | | |
| | 4 | 5 | 6 |
| Polyethylene glycol 400 | 40 | 48.2 | 42.7 |
| Glycerol | 6 | 6. | 6 |
| Behenyl alcohol | 8 | | |
| Dextrin (Pinedex #1, product of Matsutani Kagaku Kogyo Co., Ltd.) | 20 | 20 | 20 |
| Acrylic acid·methacrylic acid copolymer (PEMULEN TR-2, product of BF Goodrich Co.) | 0.5 | | |
| Hydroxypropyl cellulose (HPC-M, product of Nippon Soda Co., Ltd.) | | 0.3 | 0.3 |
| Silica gel (Aerosil 200, product of Nippon Aerosil Co., Ltd.) | | 0.5 | |
| Methyl polysiloxane (Silicone KF96A 6CS, product of Shin-Etsu Silicone Co., Ltd.) | 5 | | 5 |
| Isopropyl palmitate (Exepearl IPP, product of Kao Corporation) | | 5 | |
| Polyoxyethylene hardened castor oil (Emanon CH40, product of Kao Corporation) | 0.5 | | |
| Polyoxyethylene·methyl polysiloxane copolymer (KF6017, product of Shin-Etsu Silicone Co., Ltd.) | | | 1 |
| Activated zeolite (Zeolam A4, product of Tosoh Corp.) | 20 | 20 | 20 |
| Sodium chloride (average particle size: 500 µm) | | | 5 |
| Effect by *gommage* | ⊚ | ⊚ | ⊚ |
| Warmed feeling | ⊚ | ⊚ | ⊚ |

### Example 4:

*Gommage* cosmetic compositions having their corresponding formulations shown in Table 4 were prepared in accordance with a method known *per se* in the art to evaluate them as to an effect by *gommage*, a warmed feeling and a foaming and cleansing effect upon their use in accordance with the same evaluation standard as in Example 1.

As a result, it is understood that when a heat-generating component is incorporated into the cosmetic compositions according to the present invention, a good warmed feeling is given in addition to the effect by *gommage* as shown in Table 4. Further, when a surfactant is incorporated into the cosmetic compositions, foamability and cleansability are also brought about.

**Table 4**

| (% by weight) | | | |
|---|---|---|---|
| | Invention product | | |
| | 7 | 8 | 9 |
| Polyethylene glycol 400 | 46.55 | 44.3 | 42.8 |
| Glycerol | 10 | 10 | 10 |
| Behenyl alcohol | 2 | | |
| Dextrin (Pinedex #1, product of Matsutani Kagaku Kogyo Co., Ltd.) | 15 | 15 | 15 |
| Hydroxypropyl cellulose (HPC-M, product of Nippon Soda Co., Ltd.) | 0.2 | 0.2 | 0.2 |
| Silica gel (Aerosil 200, product of Nippon Aerosil Co., Ltd.) | 0.3 | | |
| Methyl polysiloxane (Silicone KF96A 1000CS, product of Shin-Etsu Silicone Co., Ltd.) | 0.5 | | |
| Isopropyl palmitate (Exepearl IPP, product of Kao Corporation) | | 0.5 | |
| Polyoxyethylene hardened castor oil (Emanon CH40, product of Kao Corporation) | 0.25 | | |
| Polyoxyethylene·methyl polysiloxane copolymer (KF6017, product of Shin-Etsu Silicone Co., Ltd.) | 0.2 | | |
| Activated zeolite (Zeolam A4, product of Tosoh Corp.) | 10 | 10 | 10 |
| Sodium cocoylisethionate | 15 | 13 | 15 |
| Cocoyldiethanolamide | | | 5 |
| Sodium chloride (average particle size: 500 µm) | | | 2 |
| Lauric acid | | 7 | |
| Effect by *gommage* | ⊚ | ⊚ | ⊚ |
| Warmed feeling | ○ | ○ | ○ |
| Foamability and cleansability | ○ | ⊚ | ⊚ |

### Industrial Applicability

The cosmetic composition of the present invention can form *gommage* on the wet skin, scarcely becomes a burden to the skin, has an excellent effect by the *gommage* formed, and gives users a pleasant feeling upon use.

## Claims

1. A *gommage* cosmetic composition comprising (A) a water-soluble polymer selected from modified starch and triglucopolysaccharide, soluble starch, dextrin, cyclodextrin, maltodextrin and hydrolysed and reduced products of starch, and (B) a substance that is liquid at 25°C (excluding water) selected from polyols, lower alcohols, liquid paraffin, silicone oil, and oils and fats, and wherein the water content of the composition is at most 5% by weight.

2. The *gommage* cosmetic composition according to Claim 1, wherein (A) the water-soluble polymer is a water-soluble polymer which forms *gommage* when a 20 % by weight solution of the water-soluble polymer in polyethylene glycol is rubbed on the skin in a state mixed with the same weight of water.

3. The *gommage* cosmetic composition according to Claim 1 or 2, wherein (A) the modified starch or triglucopolysaccharide have a weight average molecular weight of 500 to 200,000.

4. The *gommage* cosmetic composition according to any one of Claims 1 to 3 , which further comprises (C) a component which generates heat upon contact with water.

5. The *gommage* cosmetic composition according to any one of Claims 1 to 4, which further comprises (D) a surfactant.

6. The *gommage* cosmetic composition according to Claim 5, wherein the component (D) is an anionic surfactant, nonionic surfactant or amphoteric surfactant.

## Patentansprüche

1. Kosmetische Abreibzusammensetzung, umfassend (A) ein wasserlösliches Polymer, ausgewählt aus modifizierter Stärke und Triglucopolysaccharid, löslicher Stärke, Dextrin, Cyclodextrin, Maltodextrin und hydrolisierten und reduzierten Produkten von Stärke, und (B) eine Substanz, die bei 25°C flüssig ist (ohne Wasser), ausgewählt aus Polyolen, niedrigen Alkoholen, flüssigem Paraffin, Silikonöl und Ölen und Fetten, und worin der Wassergehalt der Zusammensetzung maximal 5 Gew.-% ist.

2. Kosmetische Abreibzusammensetzung nach Anspruch 1, worin (A) das wasserlösliche Polymer ein wasserlösliches Polymer ist, das eine Gummierung bildet, wenn eine 20 Gew.-%ige Lösung des wasserlöslichen Polymers in Polyethylenglycol auf der Haut in einem Zustand gerieben wird, dass es mit dem gleichen Gewicht an Wasser vermischt ist.

3. Kosmetische Abreibzusammensetzung nach Anspruch 1 oder 2, worin (A) die modifizierte Stärke oder Triglucopolysaccharid ein Molekulargewicht im Gewichtsmittel von 500 bis 200.000 haben.

4. Kosmetische Abreibzusammensetzung nach einem der Ansprüche 1-3, die weiterhin (C) eine Komponente enthält, die Wärme beim Kontakt mit Wasser erzeugt.

5. Kosmetische Abreibzusammensetzung nach einem der Ansprüche 1-4, die weiterhin (D) ein Tensid enthält.

6. Kosmetische Abreibzusammensetzung nach Anspruch 5, worin die Komponente (D) ein anionisches, nichtionisches oder amphoteres Tensid ist.

## Revendications

1. Composition cosmétique de gommage comprenant (A) un polymère soluble dans l'eau choisi parmi l'amidon et le triglucopolysaccharide modifiés, l'amidon soluble, la dextrine, la cyclodextrine, la maltodextrine et les produits d'amidon hydrolysés et réduits, et (B) une substance qui est liquide à 25°C (à l'exclusion de l'eau) choisie parmi les polyols, les alcools inférieurs, la paraffine liquide, une huile de silicone et les huiles et les matières grasses, et dans laquelle la teneur en eau de la composition est d'au plus 5 % en poids.

2. Composition cosmétique de gommage selon la revendication 1, dans laquelle (A) le polymère soluble dans l'eau est un polymère soluble dans l'eau qui forme un gommage lorsqu'une solution à 20 % en poids du polymère soluble dans l'eau dans du polyéthylèneglycol est frottée sur la peau dans un état mélangé avec le même poids d'eau.

3. Composition cosmétique de gommage selon la revendication 1 ou 2, dans laquelle (A) l'amidon ou triglucopolysaccharide modifié a une masse moléculaire moyenne en poids de 500 à 200 000.

4. Composition cosmétique de gommage selon l'une quelconque des revendications 1 à 3, qui comprend en outre (C) un composant qui génère de la chaleur au contact avec de l'eau.

5. Composition cosmétique de gommage selon l'une quelconque des revendications 1 à 4, qui comprend en outre (D) un tensioactif.

6. Composition cosmétique de gommage selon la revendication 5, dans laquelle le composant (D) est un tensioactif anionique, un tensioactif non ionique ou un tensioactif amphotère.
